# EUROPEAN PATENT SPECIFICATION

(11) **EP 1 737 819 B1**
(45) Date of publication and mention of the grant of the patent: **22.12.2010**
(21) Application number: 05716336.2
(22) Date of filing: 23.03.2005
(51) Int. Cl.: C07D 207/16

(54) **AMINO ACID AND PEPTIDE CONJUGATES OF ARYLALKYLIC ACIDS FOR COSMETIC USE**
AMINOSÄURE- UND PEPTID-KONJUGATE VON ARYLALKYLSÄUREN ZUR VERWENDUNG IN DER KOSMETIK
AMINOACIDE ET PEPTIDE CONJUGUÉS D'ACIDE ARYL-ALKYLE DESTINES A ETRE UTILISES EN COSMETIQUE

(30) Priority: 26.03.2004 EP 04007282
(43) Date of publication of application: 03.01.2007
(73) Proprietor: DSM IP Assets B.V., 6411 TE Heerlen (NL)
(72) Inventor: KLOCK, Jochen, 79104 Freiburg (DE); MAILLAN, Philippe Emmanuel, F-68440 Eschentzwiller (FR); VOLLHARDT, Juergen H., CH-4433 Ramlinsburg (CH); BEUMER, Raphael, 79541 Loerrach (DE)
(74) Representative: Best, Michael
(86) International application number: PCT/EP2005/003116
(87) International publication number: WO 2005/092850

(56) References cited:
- EP-A- 0 443 559
- WO-A-97/18235
- WO-A-97/18239
- KOIKOV ET AL.: "Sub-Nanomolar hMC1R Agonists by End-Capping of the Melanocortin Tetrapeptide His-D-Phe-Arg-Trp-NH2" BIOORG. MED. CHEM. LETT., vol. 13, 2003, pages 2647-2650, XP002342336
- SHIOSAKI ET AL.: "Development of Potent and Selective CCK-A Receptor Agonists from Boc-CCK-4: Tetrapetides Containing Lys(N?)-Amide Residues" J. MED. CHEM., vol. 35, 1992, pages 2007-2014, XP002342337
- GUENTHER R ET AL: "Protease Catalysis Mediated by a Substrate Mimetic: A Novel Enzymatic Aproach to the Synthesis of Carboxylic Acid Amides" CHEMISTRY - A EUROPEAN JOURNAL, WILEY - V C H VERLAG GMBH & CO. KGAA, WEINHEIM, DE, vol. 6, no. 3, 1 January 2000 (2000-01-01) , pages 463-467, XP002206637 ISSN: 0947-6539
- QIANG LU ET AL: "Zn2+ -CHELATING MOTIF-TETHERED SHORT-CHAIN FATTY ACIDS AS NOVEL CLASS OF HISTONE DEACETYLASE INHIBITORS" JOURNAL OF MEDICINAL CHEMISTRY, AMERICAN CHEMICAL SOCIETY, WASHINGTON, US, vol. 47, no. 2, 11 December 2003 (2003-12-11), pages 467-474, XP002375296 ISSN: 0022-2623
- LEONE-BAY A ET AL: "4-Ú4-Ú(2-HYDROXYBENZOYL)AMINO 3/4 PHENYL 3/4 BUTYRIC ACID AS A NOVEL ORAL DELIVERY AGENT FOR RECOMBINANT HUMAN GROWTH HORMONE" JOURNAL OF MEDICINAL CHEMISTRY, AMERICAN CHEMICAL SOCIETY, WASHINGTON, US, vol. 39, 21 June 1996 (1996-06-21), pages 2571-2578, XP002914793 ISSN: 0022-2623
- GRAHAM D W ET AL: "INHIBITION OF THE MAMMALIAN BETA-LACTAMASE RENAL DIPEPTIDASE (DEHYDROPEPTIDASE-I) BY (Z)-2-(ACYLAMINO)-3-SUBSTITUTED-PROPENOIC ACIDS" JOURNAL OF MEDICINAL CHEMISTRY, AMERICAN CHEMICAL SOCIETY, WASHINGTON, US, vol. 30, 1 January 1987 (1987-01-01), pages 1074-1090, XP001064529 ISSN: 0022-2623
- PRASIPAN N ET AL: "N-1 AND C-2 SUBSTITUTED TRYPTOPHANS AS POTENTIAL INHIBITORS OF SICKLE CELL HEMOGLOBIN GELATION" JOURNAL OF HETEROCYCLIC CHEMISTRY, HETEROCORPORATION. PROVO, US, vol. 29, no. 2, 1 March 1992 (1992-03-01), pages 335-341, XP000984090 ISSN: 0022-152X

## Description

The present invention is directed to topical cosmetic compositions containing amino acid- or peptide conjugates with short chain Arylalkyl carboxylic acids. It was found that the specific peptide or amino acid conjugates in particular the conjugates with phenylbutyric acid are particularly useful for treating wrinkles but also for thickening the epidermis, repairing the skins lipid barrier, for protection against hair loss and for improving hair growth.

Human skin undergoes certain normal cornification processes which give the skin its characteristic appearance. Casual factors or external factors such as a raw climate, wind, photo-damage and irritation triggered by the sun, rain and snow, however, disturb this normal condition of the skin, and there appears a roughness, a formation of scales (for example on the scalp), an excessive keratinization and similar phenomena. Furthermore, in the course of aging of the skin various signs appear that are especially reflected by a change in the structure and function of the skin. One of these signs is the appearance of fine lines and deep wrinkles, the size and number of which increases with age. The microrelief of the skin becomes less uniform and is of unisotropic nature. In parallel with age the skin becomes more sensitive towards disturbing influences, either intrinsic or extrinsic, which may result in itch, redness or even darker spots, particular on hands and the facial area due to pigmentation disorders. These unwanted signs may lead to an undesired age judgment of a person.

Cosmetic preparations are essentially useful for skin care. One aim of skin care in the cosmetic sense is to strengthen or rebuild the skin's natural function as a barrier against environmental influences (e.g. UV-light, dirt, chemicals, microorganisms) and against the loss of endogenous substances (e.g. water, natural lipids, electrolytes). If this function becomes impaired, increased resorption of toxic or allergenic substances or attack by microorganisms may result, leading to toxic or allergic skin reactions.

Another aim of skin care is to compensate for the loss by the skin of lipids and water caused by daily washing. This is particularly important, if the natural regeneration ability is inadequate. Furthermore, skin care products should protect against environmental influences, in particular against sun and wind, and delay skin aging.

Strengthening or thickening of the epidermis together with an optimized skin barrier lipid synthesis can rebuild the skin's barrier ability and is therefore of significant cosmetic value. Reduced transepidermal water loss (TEWL) is a sign of an intact lipid barrier, which acts also as first defense line to protect against the appearance of skin wrinkles.

Another strategy to fight wrinkles is to stimulate the collagen synthesis in the dermis. A number of degenerative processes act on the collagen matrix and is triggered by extrinsic factors like UV radiation, pollution in general and particular cigarette smoke or intrinsic factors leading to any chronic or subchronic inflammation. Destruction and/or impaired repair efficacy leads to a denser and less elastic macro structure of the dermis, which in turn leads to the formation of deep wrinkles. Enhancing the de novo synthesis of collagen or other structural proteins of the dermis is considered a valuable therapy to reduce the existing wrinkles and to protect against the appearance of new wrinkles.

Of particular importance for anti-aging cosmetics is to inhibit the senescence of skin cells in order to keep their regular metabolic level on a constant and beneficial level.

Hair loss or alopecia is a common affliction of humans. The most common form of hair loss in both males and females is patterned baldness or androgenic alopecia.

Hair follicles range in size from small, superficial, vellus follicles to large, deep, terminal follicles. The cyclic growth phases of hair follicles are telogen (resting), anagen I-III (developing), anagen IV-VI (growing) and catagen (involuting).

In the development of androgenic alopecia there is the gradual diminution of follicle size, with conversion of large, terminal follicles, producing thick, pigmented hair fibers (terminal hair) to small vellus follicles producing fine, non-pigmented hair fibers (vellus hair). In addition, the proportion of growing anagen follicles declines.

There exists a wide variety of literature regarding cosmetic preparations, in particular regarding cosmetic preparations for treating wrinkles and for promoting hair growth. As examples of the extensive literature it can be referred e.g. to GB 906,000, EP-A 699 429 or WO 03/086342.

WO 98/17273 discloses butyric acid derivatives for protecting against hair loss particularly in cancer patients undergoing chemotherapy and/or radiation therapy. Various butyric acid derivatives are mentioned, but the document does not disclose any peptide or amino acid conjugates of those acids.

EP 1297830 discloses various alpha- or beta-amino acid derivatives for prevention and treatment of tissue damage by ozone; however the document is silent in terms of amides at the alpha or beta-nitrogen atom with aryl containing carboxylic acids.

WO 00/15188 reports about specific peptides for the healing, hydrating and improving skin appearance as well as treatment of skin aging. For enhancing the lipophilicity the N-terminal amine is supposed to carry a fatty acid chain with 2 to 22 carbons. Aromatic carboxylic acids are not mentioned. The fatty acid residue should make the molecule more lipophilic.

EP 0864563 discloses the use of N-Acyl-hydroxyamino acid esters for protection of skin and hair by designing biomimetic compounds of ceramids. Ceramids contribute significantly to the skin lipid barrier and require at two long chain fatty acid one of them having preferentially more than 16 carbon atoms. The document does not disclose amides with aryl-containing carboxylic acids.

EP 1159952 suggest cosmetics containing Hydroxyproline or acylated Hydroxyproline. The document does not disclose aromatic carboxylic acids derivatives of amino acids or peptides.

US 5492894 discloses compositions with tripeptides up to hexapeptides to treat skin wrinkles. The peptides can optionally be modified by various substitution patterns at N- as well as the C-terminus. Arylalkoyl residues at the N-terminus are not disclosed.

WO 97/18235 and WO 97/18239 both claim peptides derived with carboxylic acids at the N-terminus and amongst others the use thereof in pharmaceutical or cosmetological compositions. However, no arylalkyl derivatives of said carboxylic acids are described. While a variety of technologies exist to prevent and to fight the signs of skin aging, to improve the appearance of the skin or to treat or prevent hair loss, there is still a demand for more efficacious ingredients.

The problem to be solved by the present invention is the provision of topical cosmetic preparations which are particularly useful for treating and/or preventing wrinkles, thickening of the epidermis, and preventing and/or treating of hair loss, but also preparations which are useful against other conditions which are observed with skin aging due to environmental or other external influences or due to age. The new topical cosmetic compositions contain compounds which should have an activity which is comparable to the activity of known cosmetically active compounds but preferably is better than the activity of the prior art compounds.

This problem is solved on the basis of the unexpected finding that certain phenylalkyl carboxylic derivatives, namely the peptide or amino acid conjugates thereof, show activity in cosmetic applications, in particular for treating and preventing wrinkles and hair loss and thickening the epidermis, but also for ameliorating the effects of aging of the skin, which may be caused by external or environmental hazards or by the natural aging of the skin.

Accordingly, the present invention provides topical cosmetic compositions comprising at least one compound represented by general formula (I) 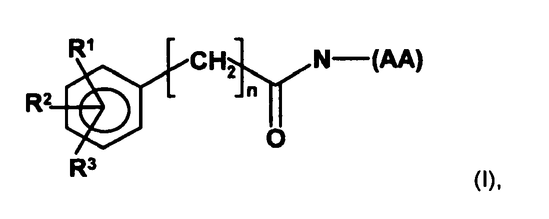 wherein R¹, R² and R³ are independently hydrogen, OR⁴, C₁-C₆ alkyl or C₂-C₆ alkenyl,
R⁴ is hydrogen, C₁-C₆ alkyl or C₂-C₆ alkenyl,
N-(AA) represents the residue of an amino acid or of a peptide which is bonded over the N-terminus of the amino acid or the peptide and the peptide is composed of 2 to 6, which means 2, 3, 4, 5 or 6, amino acids and
n=3,
and a cosmetically acceptable excipient or diluent.

The C-terminus of the amino acid- or peptide residues can be unmodified or esterified with a C₁-C₁₆ hydrocarbon moiety. Preferred is a modification, if necessary, leading to a partition coefficient of log p(Octanol/Water) from 0 to 6, preferably of from 0 to 4.

According to the present invention compounds are preferred which have a partition coefficient of log p(Octanol/Water) from 0 to 6, preferably from 0 to 4. If the C-terminus of the amino acid- or peptide residue is esterified with a C₁-C₁₆ hydrocarbon residue, the residue is preferably selected to provide the above partition coefficient. The C₁-C₁₆ hydrocarbon residue is preferably an aliphatic residue, more preferably a C₁-C₁₆- (preferably C₁-C₆-) alkyl or C₂-C₁₆- (preferably C₂-C₆-) alkenyl residue which can be straight chained or branched. Furthermore, residues comprising an aliphatic C₃-C₈ ring structure are preferred.

In a more preferred embodiment R¹ and R² are both hydrogen and R³ is a residue -O-C₁-C₆ alkyl, in particular a methoxy residue.

In a further preferred embodiment all residues R¹, R² and R³ are hydrogen.

Particularly preferred are those embodiments, wherein N-(AA) represents the residue of the amino acids Glycine, α- or β-Alanine, Valine, Leucine, Isoleucine, Proline, Phenylalanine, Tryptophan, Methionine, Selenomethionine, Serine, Threonine, Cysteine, Hydroxyproline, Asparagin, Glutamine, Aspartic acid, Glutamic acid, Lysine, Hydroxylysine, Histidine, Arginine, Ornithine, Citrulline, Taurine, Sarcosine and Statine, Norleucine, Norvaline, or 2-N-Methylnorleucine. Particular suitable are the natural isomers of the mentioned amino acids.

More preferred amino acids are Isoleucine, Arginine, Hydroxyproline. Most preferred is Hydroxyproline, preferably with the modification of having an ethyl or palmityl ester group on the C-terminus.

Preferred are also embodiments, wherein N-(AA) represents the residue of a peptide selected from Carnosine (β-Ala-His), Homocarnosine, Balenine, Anserine, Phe-β-Ala = Aspartame, Arg-Pro or Pro-Arg, Gln-β-Ala-His Glutathione (γ-Glu-Cys-Gly), Lys-Gly His, Lys Thr Ser, Leu-Arg-Trp, Ile-Lys-Trp and Leu-Lys-Trp, Gly-Pro-Tyr, lysine-proline-valine, Arg-Lys-Arg, Arg-Gly-Asp, X1-X2-Pro, X1-X2-HPro, Arg-Gly-Asp-Ser, Gly-Gln-Pro-Arg. Phe-Gly-Ala-Leu-H, PhBu- Gly-Gln-Pro-Arg, Arg-Pro-Phe-Phe, tuftsine (Tyr-Lys-Pro-Arg), regine (Gly-Gln-Pro-Arg), Phe-Tyr-Arg-Pro-Arg, Ala-Arg-Asp-Pro-Arg, Asn-Ser-Leu-Asp-Phe, Lys-Thr-Thr-Lys-Ser, Leu-Arg-Gly-Ile-Leu, Lys-Gly-Ile-Leu, Lys-Leu-Asp-Ala-Pro-Thr. Particular preferred are dipeptides and tripeptides such as X1-Gly-Pro, X1-Pro-Pro, Gly-Pro-Ala, Gly-Ala-Pro. X1 and X2 means any one of the above-identified amino acids, in particular the natural occurring amino acids.

The compounds of the topical cosmetic compositions of the present invention, i.e. the conjugates of the amino acids or peptides with arylalkyl carboxylic acids can be used alone or in mixtures. While the peptides which are useful for preparing the compounds used in the topical cosmetic compositions of the present invention can be made using a direct synthesis method they can also be made by protein degradation. In case of using a protein hydrolysis process the resulting mixture can be used to make the conjugate and the resulting product would also be suitable according to this invention. However, the preferred embodiment is to use mixtures of not more than 3 compounds more preferred is the use of only one compound.

In case that the compounds of formula I bear one or more chiral centers the compounds represented by general formula (I) may be present in a racemic mixture, in a mixture of diastereomers or in excess of an enantiomer and/or diastereomer. If one or more chiral centers are present the optical purity of the mixture is preferably ≥ 80% ee, more preferably ≥ 90% ee, most preferably ≥ 95% de. If two or more chiral centers are present the purity of the mixture is preferably ≥ 80% de, more preferably ≥ 90% de, most preferably ≥ 95% de.

The compositions of the present invention are cosmetic compositions or cosmetic preparations.

The term "cosmetic preparation" or "cosmetic composition" as used in the present application refers to cosmetic compositions as defined under the heading "Kosmetika" in Römpp Lexikon Chemie, 10th edition 1997, Georg Thieme Verlag Stuttgart, New York.

The compositions of the present invention contain the compound represented by general formula (I) with cosmetically acceptable excipients or diluents. If nothing else is stated, the excipients, additives, diluents, etc. mentioned in the following are suitable for cosmetic compositions.

If nothing else is stated, in this application parts and percentages are per weight and are based on the weight of the composition.

The compositions of the present invention are topical compositions, such as liquid or solid oil-in-water emulsions, water-in-oil emulsions, multiple emulsions, microemulsions, PET-emulsions, bickering emulsions, hydrogels, alcoholic gels, lipogels, one or multiphase solutions, foams, ointments, plasters, suspensions, powders, crèmes, cleanser, soaps and other usual compositions, which can also be applied by pens, as masks or as sprays.

The compositions of the invention can also contain usual cosmetic adjuvants and additives, such as preservatives/ antioxidants, fatty substances/ oils, water, organic solvents, silicones, thickeners, softeners, emulsifiers, sunscreens, cosmetic actives antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, e.g. those suited for providing a photoprotective effect by physically blocking out ultraviolet radiation, or any other ingredients usually formulated into cosmetics. A good overview of suitable additives for cosmetic compositions can also be found e.g. in WO 03/082232. The additives disclosed in this document, in particular the waxes, thickeners, structuring agents, film forming agents and conditioning ingredients are also suitable for the compositions of the present invention and included herein by reference. Of course, the stabilizing compositions disclosed in this document can also be used for preparing the compositions of the present invention.

The composition of the present invention can also contain one or more additional pharmaceutically or cosmetically active ingredients, in particular for preventing or reducing acne, wrinkles, lines, atrophy, inflammation, as well as topical anesthetics, artificial tanning agents and accelerators, antimicrobial agents, and antifungal agents and sunscreening additives.

Examples are peptides (e.g., Matrixyl^{™} [pentapeptide derivative]), farnesol, bisabolol, phytantriol, glycerol, urea, guanidine (e.g., amino guanidine); vitamins and derivatives thereof such as ascorbic acid, vitamin A (e.g., retinoid derivatives such as retinyl palmitate or retinyl propionate), vitamin E (e.g., tocopherol acetate), vitamin B₃ (e.g., niacinamide) and vitamin B₅ (e.g., panthenol) and the like and mixtures thereof, wax-based synthetic peptides (e.g., octyl palmitate and tribehenin and sorbitan isostearate and palmitoyl-oligopeptide), anti-acne medicaments (resorcinol, salicylic acid, and the like); antioxidants (e.g., phytosterols, lipoic acid); flavonoids (e.g., isoflavones, phytoestrogens); skin soothing and healing agents such as aloe vera extract, allantoin and the like; chelators and sequestrants; and agents suitable for aesthetic purposes such as essential oils, fragrances, skin sensates, opacifiers, aromatic compounds (e.g., clove oil, menthol, camphor, eucalyptus oil, and eugenol), desquamatory actives, anti-acne actives, vitamin B₃ compounds, anti-oxidants, peptides, hydroxy acids, radical scavengers, chelators, farnesol, anti-inflammatory agents, topical anesthetics, tanning actives, skin lightening agents, anti-cellulite agents, flavonoids, antimicrobial actives, and antifungal actives, in particular bisabolol, alkyldiols such as 1,2-pentandiol, hexanediol or 1,2-octanediol, vitamins, panthenol, phytol, phytantriol, ceramides and pseudoceramides, amino acids and bioactive peptides, protein hydrolysates, AHA acids, polyunsaturated fatty acids, plant extracts, DNA or RNA and their fragmentation products or carbohydrates, biotin, lipoic acid, conjugated fatty acids, carnitin, vitamin E, A, C, B3, B6, B12, panthenol, K1, phytantriol, oligopeptides, carnosin, biochinonen, phytofluen, phytoen, folic acid, and their corresponding derivatives.

Additionally the composition of the present invention may contain UV-A and UV-B filters. Examples of UV-B or broad spectrum screening agents, i.e. substances having absorption maximums between about 290 and 340 nm, which are preferred for combination with the compounds of the present invention, are the following organic and inorganic compounds:

Acrylates such as 2-ethylhexyl 2-cyano-3,3-diphenylacrylate (octocrylene, PARSOL® 340), ethyl 2-cyano-3,3-diphenylacrylate and the like;
- -- Camphor derivatives such as 4-methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like;
- -- Cinnamate derivatives such as octyl methoxycinnamate (PARSOL® MCX), ethoxyethyl methoxycinnamate, diethanolamine methoxycinnamate (PARSOL® Hydro), isoamyl methoxycinnamate and the like as well as cinnamic acid derivatives bond to siloxanes;
- -- p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate,
- -- Benzophenones such as benzophenone-3, benzophenone-4, 2,2', 4, 4'-tetrahydroxy-benzophenone, 2,2'-dihydroxy-4,4'-dimethoxybenzophenone and the like;
- -- Esters of Benzalmalonic acid such as di-(2-ethylhexyl) 4-methoxybenzalmalonate;
- -- Esters of 2-(4-ethoxy-anilinomethylene)propandioic acid such as 2-(4-ethoxy anilinomethylene)propandioic acid diethyl ester as described in the European Patent Publication EP 0895 776;
- -- Organosiloxane compounds containing benzmalonate groups as described in the European Patent Publications EP 0358584 B1, EP 0538431 B1 and EP 0709080 A1;
- -- Drometrizole trisiloxane (Mexoryl XL);
- -- Pigments such as microparticulated TiO₂, and the like. The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.
- -- Imidazole derivatives such as e.g. 2-phenyl benzimidazole sulfonic acid and its salts (PARSOL®HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like.
- -- Salicylate derivatives such as isopropylbenzyl salicylate, benzyl salicylate, butyl salicylate, octyl salicylate (NEO HELIOPAN OS), isooctyl salicylate or homomenthyl salicylate (homosalate, HELIOPAN) and the like.
- -- Triazine derivatives such as octyl triazone (UVINUL T-150), dioctyl butamido triazone (UVASORB HEB), bis ethoxyphenol methoxyphenyl triazine (Tinosorb S) and the like.
- -- Encapsulated UV-filters such as encapsulated octyl methoxycinnamate (Eusolex UV-pearls) and the like.

Examples of broad spectrum or UV A screening agents i.e. substances having absorption maximums between about 320 and 400 nm, which are preferred for combination with the compounds of the present invention are the following organic and inorganic compounds:
- -- Dibenzoylmethane derivatives such as 4-tert. butyl-4'-methoxydibenzoylmethane (PARASOL® 1789), dimethoxydibenzoylmethane, isopropyldibenzoylmethane and the like;
- -- Benzotriazole derivatives such as 2,2'-methylene-bis-(6-(2H-benzotriazole-2-yl)-4-(1,1,3,3,-tetramethylbutyl)-phenol (TINOSORB M) and the like;
- -- phenylene-1,4-bis-benzimidazolsulfonic acids or salts such as 2,2-(1,4-phenylene)bis-(1H-benzimidazol-4,6-disulfonic acid) (Neoheliopan AP);
- -- amino substituted hydroxybenzophenones such as 2-(4-Diethylamino-2-hydroxy-benzoyl)-benzoic acid hexylester as described in the European Patent Publication EP 1046391;
- -- Pigments such as microparticulated ZnO or TiO₂ and the like. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.

As dibenzoylmethane derivatives have limited photostability it may be desirable to photostabilize these UV-A screening agents. Thus, the term "conventional UV-A screening agent" also refers to dibenzoylmethane derivatives such as e.g. PARSOL® 1789 stabilized by, e.g.,
- -- 3,3-Diphenylacrylate derivatives as described in the European Patent Publications EP-A 0 514 491 and EP-A 0 780 119;
- -- Benzylidene camphor derivatives as described in the US Patent No. 5,605,680;
- -- Organosiloxanes containing benzmalonate groups as described in the European Patent Publications EP-A 0358584, EP-A 0538431 and EP-A 0709080.

A good overview of UV-A and UV-B-filters which can be added to the compositions of the present invention can also be found in DE-A 103 27 432. All UV-filter compounds disclosed in this document are also useful as components for the compositions of the present invention and are included herein by reference.

The compositions of the present invention preferably contain one or more antioxidants/preservatives. Based on the invention all known antioxidants usually formulated into cosmetics can be used. Especially preferred are antioxidants chosen from the group consisting of amino acids (e.g. glycine, histidine, tyrosine, tryptophan) and their derivatives, imidazole (e.g. urocanic acid) and derivatives, peptides such as D,L-carnosine, D-carnosine, L-carnosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cysteine, cystine, cystamine and its glycosyl-, N-acetyl-, methyl-, ethyl-, propyl-, amyl-, butyl- and lauryl-, palmitoyl-; oleyl-, y-linoleyl-, cholesteryl- and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinsulfoximine, buthioninsulfone, penta-, hexa-, heptathioninsulfoximine) in very low compatible doses (e.g. pmol to µmol/kg), additionally (metal)-chelators (such as α-hydroxyfatty acids, palmic-, phytinic acid, lactoferrin), α-hydroxyacids (such as citric acid, lactic acid, malic acid), huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, vitamin C and derivatives (such as ascorbylpalmitate and ascorbyltetraisopalmitate, Mg-ascorbylphosphate, Na-ascorbylphosphate, ascorbylacetate, ascorbylglucoside), tocopherol and derivates (such as vitamin-E-acetate), mixtures of nat. vitamin E, vitamin A and derivatives (vitamin-A-palmitate and -acetate) as well as coniferylbenzoate, rutinic acid and derivatives, α-glycosylrutin, ferulic acid, furfurylidenglucitol, carnosin, butylhydroxytoluene, butylhydroxyanisole, trihydroxybutyrophenone, urea and its derivatives, mannose and derivatives, zinc and derivatives (e.g. ZnO, ZnSO₄), selenium and derivatives (e.g. selenomethionine), stilbenes and derivatives (such as stilbenoxide, trans-stilbenoxide) and suitable derivatives (salts, esters, ethers, sugars, nucleotides, nucleosides, peptides and lipids) of the named active ingredients. One or more preservatives/antioxidants may be present in an amount about 0.01 wt.% to about 10 wt.% of the total weight of the composition of the present invention. Preferably, one or more preservatives/antioxidants are present in an amount about 0.1 wt.% to about 1 wt.%.

Typically topical formulations also contain surface active ingredients like emulsifiers, solubilizers and the like. An emulsifier enables two or more not miscible components to be combined homogeneously. Moreover, the emulsifier acts to stabilize the composition. Emulsifiers that may be used in the present invention in order to form O/W, W/O, O/W/O or W/O/W emulsions/microemulsions include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polyglyceryl-4 oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further suitable emulsifiers are phosphate esters and the salts thereof such as cetyl phosphate (Amphisol^{®} A), diethanolamine cetyl phosphate (Amphisol^{®}), potassium cetyl phosphate (Amphisol^{®} K), sodium glyceryl oleate phosphate, hydrogenated vegetable glycerides phosphate and mixtures thereof. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof. The preferred emulsifiers are cetyl phosphate (Amphisol^{®} A), diethanolamine cetyl phosphate (Amphisol^{®}), potassium cetyl phosphate (Amphisol^{®} K), PVP Eicosene copolymer, acrylates/C₁₀₋₃₀-alkyl acrylate crosspolymer, PEG-20 sorbitan isostearate, sorbitan isostearate, and mixtures thereof. The one or more emulsifiers are present in a total amount about 0.01 wt.% to about 20 wt.% of the total weight of the composition of the present invention. Preferably, about 0.1 wt.% to about 10 wt.% of emulsifiers are used.

The lipid phase of the topical compositions can advantageously be chosen from:
- mineral oils and mineral waxes;
- oils such as triglycerides of caprinic acid or caprylic acid, preferable castor oil;
- oils or waxes and other natural or synthetic oils, in an preferred embodiment esters of fatty acids with alcohols e.g. isopropanol, propylene glycol, glycerin or esters of fatty alcohols with carboxylic acids or fatty acids;
- alkylbenzoates; and/or
- silicone oils such as dimethylpolysiloxane, diethylpolysiloxane, diphenylpolysiloxane, cyclomethicones
and mixtures thereof.

Exemplary fatty substances which can be incorporated in the oil phase of the emulsion, micro-emulsion, oleo gel, hydrodispersion or lipodispersion of the present invention are advantageously chosen from esters of saturated and/or unsaturated, linear or branched alkyl carboxylic acids with 3 to 30 carbon atoms, and saturated and/or unsaturated, linear and/or branched alcohols with 3 to 30 carbon atoms as well as esters of aromatic carboxylic acids and of saturated and/or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexyllaureate, n-decyloleat, isooctylstearate, isononylstearate, isononylisononanoate, 2-ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as synthetic, half-synthetic or natural mixtures of such esters e.g. jojoba oil.

Other fatty components suitable for use in the topical compositions of the present invention include polar oils such as lecithins and fatty acid triglycerides, namely triglycerol esters of saturated and/or unsaturated, straight or branched carboxylic acid with 8 to 24 carbon atoms, preferably of 12 to 18 carbon-atoms whereas the fatty acid triglycerides are preferably chosen from synthetic, half synthetic or natural oils (e.g. cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalane and squalene, polyolefins, hydrogenated polyisobutenes and isohexadecanes, favored polyolefins are polydecenes; dialkyl ethers such as dicaprylylether; linear or cyclic silicone oils such as preferably cyclomethicone (octamethylcyclotetrasiloxane; cetyldimethicone, hexamethylcyclotrisiloxane, polydimethylsiloxane, poly(methylphenylsiloxane) and mixtures thereof.

Other fatty components which can advantageously be incorporated in topical compositions of the present invention are isoeikosane; neopentylglycoldiheptanoate; propyleneglycol-dicaprylate/ dicaprate; caprylic/capric/diglycerylsuccinate; butyleneglycol caprylat/caprat; C₁₂₋₁₃-alkyllactate; di-C₁₂₋₁₃ alkyltartrate; triisostearin; dipentaerythrityl hexacaprylat-/hexacaprate; propylenglycolmonoisostearate; tricaprylin; dimethylisosorbid. Especially beneficial is the use of mixtures C₁₂₋₁₅-alkylbenzoate and 2-ethylhexylisostearate, mixtures C₁₂₋₁₅-alkylbenzoate and isotridecylisononanoate as well as mixtures of C₁₂₋₁₅-alkylbenzoate, 2-ethylhexylisostearate and isotridecylisononanoate.

The oily phase of the compositions of the present invention can also contain natural vegetable or animal waxes such as bee wax, china wax, bumblebee wax and other waxes of insects as well as shea butter and cocoa butter.

A moisturizing agent may be incorporated into a topical composition of the present invention to maintain hydration or rehydrate the skin. Moisturizers that prevent water from evaporating from the skin by providing a protective coating are called emollients. Additionally an emollient provides a softening or soothing effect on the skin surface and is generally considered safe for topical use. Preferred emollients include mineral oils, lanolin, petrolatum, capric/caprylic triglyceraldehydes, cholesterol, silicones such as dimethicone, cyclomethicone, almond oil, jojoba oil, avocado oil, castor oil, sesame oil, sunflower oil, coconut oil and grape seed oil, cocoa butter, olive oil aloe extracts, fatty acids such as oleic and stearic, fatty alcohols such as cetyl and hexadecyl (ENJAY), diisopropyl adipate, hydroxybenzoate esters, benzoic acid esters of C₉₋₁₅-alcohols, isononyl iso-nonanoate, ethers such as polyoxypropylene butyl ethers and polyoxypropylene cetyl ethers, and C₁₂₋₁₅-alkyl benzoates, and mixtures thereof. The most preferred emollients are hydroxybenzoate esters, aloe vera, C₁₂₋₁₅-alkyl benzoates, and mixtures thereof. An emollient is present in an amount of about 1 wt.% to about 20 wt.% of the total weight of the composition. The preferred amount of emollient is about 2 wt.% to about 15 wt.%, and most preferably about 4 wt.% to about 10 wt.%.

Moisturizers that bind water, thereby retaining it on the skin surface are called humectants. Suitable humectants can be incorporated into a topical composition of the present invention such as glycerin, polypropylene glycol, 1,2-pentandiol, polyethylene glycol, lactic acid, pyrrolidone carboxylic acid, urea, phospholipids, collagen, elastin, ceramides, lecithin sorbitol, PEG-4, and mixtures thereof. Additional suitable moisturizers are polymeric moisturizers of the family of water soluble and/ or swellable/ and/ or with water gelating polysaccharides such as hyaluronic acid, chitosan and/or a fucose rich polysaccharide which is e.g. available as Fucogel®1000 (CAS-Nr. 178463-23-5) by SOLABIA S. One or more humectants are optionally present at about 0.5 wt.% to about 8 wt.% in a composition of the present invention, preferably about 1 wt.% to about 5 wt.%.

The aqueous phase of the topical compositions of the present invention can contain the usual cosmetic or pharmaceutical additives such as alcohols, especially lower alcohols, preferably ethanol and/ or isopropanol, low diols or polyols and their ethers, preferably propyleneglycol, glycerin, ethyleneglycol, ethyleneglycol monoethyl- or monobutylether, propyleneglycol monomethyl- or -monoethyl- or -monobutylether, diethyleneglycol monomethyl- or -monoethylether and analogue products, polymers, foam stabilizers; electrolytes and especially one or more thickeners. Thickeners that may be used in formulations of the present invention to assist in making the consistency of a product suitable include carbomer, siliciumdioxide, magnesium and/ or aluminum silicates, beeswax, stearic acid, stearyl alcohol polysaccharides and their derivatives such as xanthan gum, hydroxypropyl cellulose, polyacrylamides, acrylate crosspolymers preferably a carbomer, such as carbopole^{®} of type 980, 981, 1382, 2984, 5984 alone or mixtures thereof. Suitable neutralizing agents which may be included in the composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginin and lysine and any combination of any foregoing. The neutralizing agent can be present in an amount of about 0.01 wt.% to about 8 wt.% in the composition of the present invention, preferably, 1 wt.% to about 5 wt.%.

The addition of electrolytes into the composition of the present invention may be necessary to change the behavior of a hydrophobic emulsifier. Thus, the emulsions/microemulsions of this invention may contain preferably electrolytes of one or several salts including anions such as chloride, sulfates, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 wt.% to about 8 wt.% in the composition of the present invention.

The topical compositions of the invention can preferably be provided in the form of a lotion, a thickened lotion, a gel, a cream, a milk, an ointment, a powder or a solid tube stick and can be optionally be packaged as an aerosol and can be provided in the form of a mousse, foam or a spray. The compositions according to the invention can also be in the form of a suspension or dispersion in solvents or fatty substances, or alternatively in the form of an emulsion or microemulsion (in particular of O/W or W/O type, O/W/O or W/O/W-type), such as a cream or a milk, a vesicular dispersion, in the form of an ointment, a gel, a solid tube stick or an aerosol mousse. The emulsions can also contain anionic, nonionic, cationic or amphoteric surfactants.

The topical application is preferably at least once per day, e.g. two or three times a day. Usually it takes at least two days until the desired effect is achieved. However, it can take several weeks or even months until the desired effect is achieved.

The amount of the topical composition which is to be applied to the skin depends on the concentration of the active ingredients in the compositions and the desired cosmetic or pharmaceutical effect. For example, application can be such that a crème is applied to the skin. A crème is usually applied in an amount of 2 mg crème/cm² skin. The amount of the composition which is applied to the skin is, however, not critical, and if with a certain amount of applied composition the desired effect cannot be achieved, a higher concentration of the active ingredients can be used e.g. by applying more of the composition or by applying compositions which contain more active ingredient.

According to the invention for preparing the compositions the active ingredients can be used as such or in an encapsulated form, for example in a liposomal form. Liposomes are preferably formed with lecithins with or without addition of sterols or phytosterols. The encapsulation of the active ingredients can be alone or together with other active ingredients.

In the topical compositions of the invention the compound of formula (I) is contained in an amount of preferably 0.0001 wt.-% to about 50 wt.-%, based on the total weight of the composition. More preferably, the compound is contained in the composition in an amount of about 0.01 wt.-% to about 20 wt.-%, more preferably in an amount of about 0.1 wt.-% to about 5 wt.-%, in particular in an amount of about 1 wt.-%, based on the total amount of the composition.

In case that the topical composition of the invention contains a further active ingredient, this further active ingredient is contained in an amount of preferably 0.0001 wt.-% to about 50 wt.-%, based on the total weight of the composition. More preferably, the further active ingredient is contained in the composition in an amount of about 0.01 wt.-% to about 20 wt.-%, more preferably in an amount of about 0.01 wt.-% to about 1 wt.-%, in particular in an amount of about 0.1 wt.-%, based on the total amount of the composition.

Regarding the kind of the topical preparation and the preparation of the topical preparations as well as for further suitable additives, it can be referred to the pertinent literature, e.g. to Novak G.A., Die kosmetischen Präparate - Band 2, Die kosmetischen Präparate - Rezeptur, Rohstoffe, wissenschaftliche Grundlagen (Verlag für Chem. Industrie H. Ziolkowski KG, Augsburg).

It is furthermore possible to provide the compositions of the present invention as oral composition, e.g. in the form of pills, tablets, capsules which may contain granules or pellets, as a liquid, oral formulation or as an additive to foodstuff as is generally known to a skilled person. Useful procedures and useful additives for preparing the oral compositions of the present invention are e.g. disclosed in the standard literature Remington: The Science and Practice of Pharmacy, Lippincot, Williams & Wilking (Editors) 2000, which is included herein by reference.

As usual additives for oral compositions, in particular for tablets, usual excipients such as micro-crystalline cellulose, sodium citrate, calcium carbonate, disodium or dipotassium phosphate, sodium or potassium phosphate, glycine, disintegration agents such as starch or alginic acid, binders such as polyvinylpyrolidone, saccharose, gelatin and gum arabicum lubricants such as magnesium stearate, sodium lauryl sulfate or talcum can be used. If the compositions are filed into gelatin capsules, usual additives for the preparation of granules are lactose or lactate as well as polyethylene glycols with a high molecular weight. Further additives and excipients as well as additives and excipients for other oral formulations and for food additives are known to a skilled person, and it can be referred to the pertinent literature such as "Grundzüge der Lebensmitteltechnik", Horst Dieter Tscheuschner (Editor), 2. Edition, Hamburg, Beers 1996.

The total content of the active ingredients in the oral compositions of the present invention is usually about 1% to 90%, preferably about 10% to 80%, e.g. about 50% or more. The application is such that the desired effect occurs and depends on the patient and the desired effect. A usual daily dosage can be in a range from about 0.1 µg/day to 50 mg/day, e.g. about 20 µg/day to 2 mg/day.

The compositions of the present invention can also be in the form of injectable compositions, in particular if the compositions are for promoting hair growth. The preparation of injectable compositions is known to a skilled person, and it can be referred to the pertinent literature, in particular to Remington already cited above.

The compounds of formula (I) can also be present as hydrates or solvates, and the hydrates and solvates of the active ingredients are also encompassed by the present invention.

The following examples exemplify the invention, but they should not be construed as limiting the invention.

### Example 1

### Preparation of 4-Hydroxy-1-(4-phenyl-butyryl)-pyrrolidine-2-carboxylic acid ethyl ester

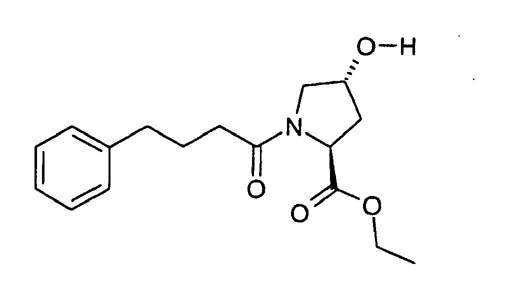

4-Hydroxy-pyrrolidine-2-carboxylic acid ethyl ester hydrochloride (13.7 g, 70.0 mmol, 1.0 eq.) was dissolved in toluene (200 mL), cooled to 10°C and NEt₃ (21.3 g, 210.0 mmol, 3.0 eq.) was added. The solution was stirred for 15 min at 10°C and 4-phenyl-butyryl chloride (12.8 g, 70.0 mmol, 1.0 eq.) in toluene (60 mL) was added. The solution was stirred over night at room temperature. The solution was washed once with an aqueous NaHCO₃ solution (10%, 100 mL)), once with H₂O (100 mL), once with Brine ((100 mL) and dried over Na₂SO₄. The solvent was evaporated under reduced pressure and the residue was purified by flash chromatography using TBME to give the product (16.7 g, 78%) as pale yellow oil. - R*_{f}* (TBME) = 0.28; ¹H NMR (CDCl₃, 300 MHz) δ = 1.19 (t, *J* = 7.1 Hz, 3H), 1.80-2.11 (m, 4H), 2.14-2.21 (m, 2H), 2.53-2.61 (m, 2H), 3.09-3.22 (m, 1H), 3.32-3.47 (m, 1H), 3.55-3.72 (m, 1H), 4.10 (q, *J* = 7.1 Hz, 2H), 4.32-4.49 (m, 2H), 7.06-7.19 (m, 5H); MS (EI) *m*/*z* = 306 (100) [M⁺+H]; IR (neat) *v*= 3399, 2938, 1738, 1620, 1434, 1184, 1083, 1030, 968.

The compound has a calculated Log POW of 2.273.

### Example 2

### Preparation of (4-Hydroxy-1-[2-(4-phenyl-butyrylamino)-acetyl]-pyrrolidine-2-carboxylic acid ethyl ester

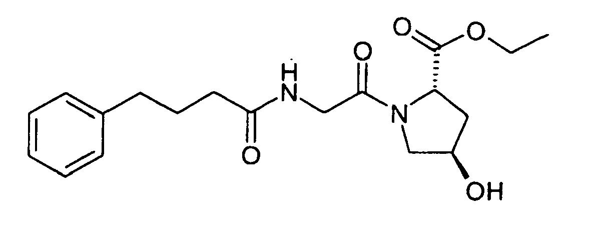

4-Phenyl-butyric acid (9.1 g, 50.0 mmol, 1.0 eq.), Glycine ethyl ester hydrochloride (7.0 g, 50.0 mmol, 1.0 eq.) and HOBt (6.6 g, 50.0 mmol, 1.0 eq.) were dissolved in DMF (1.0 L) and CH₂Cl₂ (500 mL) and cooled to 0°C. EDC (9.6 g, 50.0 mmol, 1.0 eq.) and NEt₃ (5.1 g, 50.0 mmol, 1.0 eq.) were added. The solution was allowed to warm up and then stirred for 72 h at room temperature. The solution was transferred to a separation funnel and ethyl acetate (1.0 L) and water (500 mL) were added. The organic layer was washed with an aqueous solution of 5 % HCl (500 mL), with a saturated aqueous solution of NaHCO₃ (500 mL), with a saturated aqueous solution of NaCl (500 mL) and then dried over Na₂SO₄. The solvent was evaporated under reduced pressure and the residue was purified by flash chromatography using ethyl acetate/hexane (1:1) to yield (4-Phenyl-butyrylamino)-acetic acid ethyl ester (8.0 g, 64 %) as a colorless oil. - R*_{f}* (ethyl acetate/hexane) = 0.42: ¹H NMR (CDCl₃, 300 MHz) δ = 1.26 (t, *J* = 7.1 Hz, 3H), 1.92-2.02 (m, 2H), 2.24 (t, *J* = 7.4 Hz, 2H), 2.65 (t, *J* = 7.4 Hz, 2H), 3.98 (d, *J* = 5.4 Hz, 2H), 4.18 (q, *J* = 7.1 Hz, 2H), 6.42 (br s, 1H), 7.16-7.29 (m, 5H).

(4-Phenyl-butyrylamino)-acetic acid ethyl ester (5.0 g, 20.0 mmol, 1.0 eq.) was dissolved in EtOH (40 mL) and NaOH (890 mg, 22.1 mmol, 1.1 eq.), dissolved in water (15 mL), was added and the solution stirred for 72 h at room temperature. The solvent was evaporated and the residue was taken up in TBME (100 mL) and cooled to 0°C. An aqueous solution of 12.5 % H₂SO₄ (20 mL) was added and the layers separated. The aqueous layer was extracted twice with TBME (100 mL). The organic layers were combined, washed with a saturated aqueous solution of NaCl (100 mL) and dried over Na₂SO₄. The solvent was evaporated to yield (4-Phenyl-butyrylamino)-acetic acid (4.3 g, 97 %) as a white solid. ¹H NMR (CDCl₃, 300 MHz) δ = 1.87-1.97 (m, 2H), 2.20 (t, *J* = 7.4 Hz, 2H), 2.60 (t, *J* = 7.4 Hz, 2H), 3.99 (d, *J* = 5.3 Hz, 2H), 5.95 (br s, 1 H), 7.09-7.23 (m, 5H).

(4-Phenyl-butyrylamino)-acetic acid (2.0 g, 9.0 mmol, 1.0 eq.) was dissolved in DMF (75 mL) and CH₂Cl₂ (125 mL). 4-Hydroxy-pyrrolidine-2-carboxylic acid ethyl ester hydrochloride (2.0 g, 9.9 mmol, 1.1 eq.) was added, cooled to -15°C and NEt₃ (1.0 g, 10.4 mmol, 1.1 eq.) and HBTU (3.9 g, 10.4 mmol, 1.15 eq.) were added. The solution was stirred for 72 h at room temperature. The solvent was evaporated under reduced pressure and the residue was taken up in ethyl acetate (400 mL) and washed twice with an aqueous solution of 5 % (200 mL), a saturated aqueous solution of NaHCO₃ (200 mL), with a saturated aqueous solution of NaCl (200 mL) and then dried over Na₂SO₄. The solvent was evaporated under reduced pressure and the residue was purified by flash chromatography using CH₂Cl₂/MeOH (9:1) to yield 4-Hydroxy-1-[2-(4-phenyl-butyrylamino)-acetyl]-pyrrolidine-2-carboxylic acid ethyl ester (1.0 g, 31%) as a colorless solid. - R*_{f}* (CH₂Cl₂/MeOH (9:1)) = 0.54; ¹H NMR (CDCl₃, 300 MHz) δ = 1.23-1.28 (m, 3H), 1.87-2.02 (m, 3H), 2.20-2.43 (m, 3H), 2.58-2.64 (m, 2H), 3.46-3.71 (m, 2H), 3.84-3.91 (m, 1H), 4.07-4.24 (m, 3H), 4.50-4.58 (m, 3H), 6.74 (br s, 1 H), 7.13-7.28 (m, 5H); ¹³C NMR (CDCl₃, 75.5 MHz) δ = 14.1, 27.1, 35.1, 35.5, 37.5, 41.9, 54.3, m58.1. 61.4, 69.9, 126.0, 128.4, 128.5, 141.3, 167.4, 172.1, 173.5; MS *m*/*z* = 363 (100) [M⁺+H].

The compound has a calculated Log POW of 1.17.

### Example 3

Preparation of 4-Hydroxy-1-[3-(4-phenyl-butyrylamino)-propionyl]-pyrrolidine-2-carboxylic acid ethyl ester

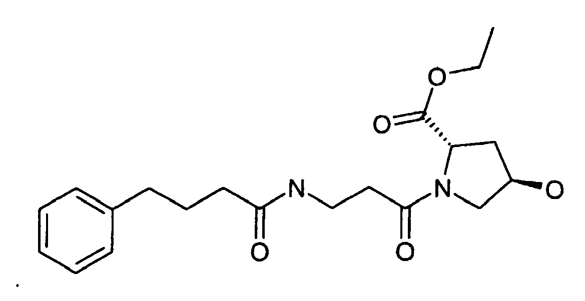

β-Alanine ethyl ester hydrochloride (14.8 g, 96.4 mmol, 1.1 eq.) was dissolved in toluene (260 mL) and cooled to 10°C. NEt₃ (5.1 g, 50.0 mmol, 1.0 eq.) was first added and then a solution of 4-Phenyl-butyric acid chloride (16.0 g, 87.6 mmol, 1.0 eq.) in toluene (50 mL). The solution was allowed to warm up and then stirred for 3 h at room temperature. The solution was concentrated under reduced pressure, ethyl acetate (150 mL) and water (200 mL) were added. The layers were separated, the organic layer was washed twice with an aqueous solution of 5% HCl (150 mL), with a saturated aqueous solution of NaHCO₃ (150 mL), with a saturated aqueous solution of NaCl (150 mL) and then dried over Na₂SO₄. The solvent was evaporated under reduced pressure and the residue was purified by flash chromatography using ethyl acetate/hexane (1:1) to yield 3-(4-Phenyl-butyrylamino)-propionic acid ethyl ester (20.5 g, 89 %) as a colorless oil. - R*_{f}* (ethyl acetate/hexane) = 0.36; ¹H NMR (CDCl₃, 300 MHz) δ = 1.20 (t, *J* = 7.2 Hz, 3H), 1.86-1.98 (m, 2H), 2.11-2.16 (m, 2H), 2.48 (t, *J* = 6.2 Hz, 2H), 2.57-2.62 (m, 2H), 3.45 (q, *J* = 6.1 Hz, 2H), 4.09 (q, *J* = 7:2 Hz, 2H), 6.47 (br s, 1 H), 7.11-7.28 (m, 5H).

3-(4-Phenyl-butyrylamino)-propionic acid ethyl ester (9.1 g, 34.7 mmol, 1.0 eq.) was dissolved in EtOH (75 mL) and NaOH (1.88 g, 46.8 mmol, 1.4 eq.), dissolved in water (25 mL), was added and the solution stirred for 16 h at room temperature. The solvent was evaporated and the residue was taken up in TBME (100 mL). An aqueous solution of 25 % H₂SO₄ (10 mL) was added and the layers were separated. The aqueous layer was extracted twice with TBME (100 mL). The organic layers were combined, washed with a saturated aqueous solution of NaCl (100 mL) and dried over Na₂SO₄. The solvent was evaporated under reduced pressure to yield 3-(4-Phenyl-butyrylamino)-propionic acid (7.3 g, 90 %) as a beige solid. ¹H NMR (CDCl₃, 300 MHz) δ = 1.77-1.87 (m, 2H), 2.05-2.11 (m, 2H), 2.41-2.53 (m, 4H), 3.37 (q, *J* = 6.0 Hz, 2H), 6.52 (t, *J* = 5.9 Hz, 1H), 7.03-7.18 (m, 5H).

3-(4-Phenyl-butyrylamino)-propionic acid (2.0 g, 8.5 mmol, 1.0 eq.) was dissolved in DMF (213 mL) and CH₂Cl₂ (100 mL). 4-Hydroxy-pyrrolidine-2-carboxylic acid ethyl ester hydrochloride (1.7 g, 8.5 mmol, 1.0 eq.) was added, cooled to 5°C and NEt₃ (1.7 g, 17.0 mmol, 2.0 eq.) and HBTU (4.2 g, 11.1 mmol, 1.3 eq.) were added. The solution stirred for 72 h at room temperature. The solvent was evaporated under reduced pressure and the residue was taken up in ethyl acetate (500 mL) and an aqueous solution of 5 % HCl (250 mL). The layers were separated and the aqueous layer was extracted with ethyl acetate (150 mL). The combined organic layers were washed with an aqueous solution of

5 % (200 mL), twice with a saturated aqueous solution of NaHCO₃ (200 mL), twice with a saturated aqueous solution of NaCl (200 mL) and then dried over Na₂SO₄. The solvent was evaporated under reduced pressure and the residue was purified by flash chromatography using CH₂Cl₂/MeOH (9:1) to yield 4-Hydroxy-1-[3-(4-phenyl-butyrylamino)-propionyl]-pyrrolidine-2-carboxylic acid ethyl ester (2.2 g, 70 %) as a slightly yellow oil. - R*_{f}* (CH₂Cl₂/MeOH (9:1)) = 0.50; ¹H NMR (CDCl₃, 300 MHz) δ = 1.16-1.22 (m, 3H), 1.79-2.57 (m, 9H), 3.30-3.78 (m, 5H), 4.01-4.16 (m, 3H), 4.38-4.49 (m, 2H), 6.37-6.42 (m, 1 H), 7.07-7.22 (m, 5H); ¹³C NMR (CDCl₃, 75.5 MHz) δ = 14.1, 27.1, 34.4, 34.9, 35.2, 35.9, 37.7, 55.3, 57.8, 61.2, 69.8, 125.9, 128.3, 128.4, 141.5, 170.9, 172.4, 173.2; MS *m*/*z* = 377 (100) [M⁺+H].

The compound has a calculated Log POW of 0.96.

### Example 4

### Preparation of 3-(3H-Imidazol-4-yl)-2-[3-(4-phenyl-butyrylamino)-propionylamino]-propionic acid ethyl ester

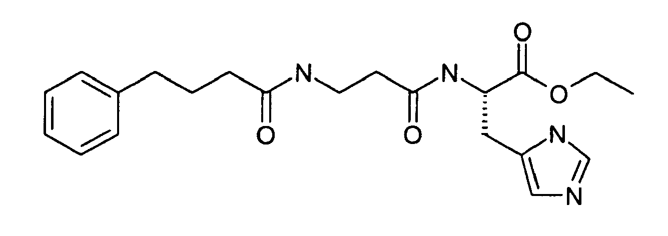

3-(4-Phenyl-butyrylamino)-propionic acid (235 mg, 1.0 mmol, 1.0 eq.) was dissolved in CH₂Cl₂ (20 mL). 2-Amino-3-(3H-imidazol-4-yl)-propionic acid ethyl ester dihydrochloride (256 g, 1.0 mmol, 1.0 eq.) was added, cooled to 5°C and NEt₃ (304 mg, 3.0 mmol, 3.0 eq.) and HBTU (455 mg, 1.2 mmol, 1.2 eq.) were added. The solution was stirred for 48 h at room temperature. The solvent was evaporated under reduced pressure and the residue was taken up in ethyl acetate (50 mL) and water (50 mL). The layers were separated and the aqueous layer was extracted with ethyl acetate (50 mL). The combined organic layers were washed twice with a saturated aqueous solution of NaHCO₃ (75 mL), twice with a saturated aqueous solution of NaCl (75 mL) and then dried over Na₂SO₄. The solvent was evaporated under reduced pressure and the residue was purified by flash chromatography using MeOH/Aceton (1:9) to yield 3-(3H-Imidazol-4-yl)-2-[3-(4-phenyl-butyrylamino)-propionylamino]-propionic acid ethyl ester (124 mg, 31 %) as a white solid. - R*_{f}* (Aceton/MeOH (9:1)) = 0.54; ¹H NMR (CDCl₃, 300 MHz) δ = 1.17 (t, *J* = 7.1 Hz, 3H), 1.80-1.90 (m, 2H), 2.06-2.11 (m, 2H), 2.34-2.40 (m, 2H), 2.55 (t, *J* = 7.6 Hz, 2H), 2.95-3.08 (m, 2H), 3.38-3.58 (m, 2H), 4.07 (q, *J* = 7.1 Hz, 2H), 4.68-4.73 (m, 1H), 6.65-6.72 (m, 2H), 7.07-7.22 (m, 7H), 7.35 (s, 1H); ¹³C NMR (CDCl₃, 75.5 MHz) δ = 13.2, 26.1, 34.3, 34.7, 34.9, 35.3, 51.6, 60.4, 76.21 124.9, 127.3, 127.5, 134.1, 140.7, 170.3, 170.7, 172.0; MS *m*/*z* = 401 (100) [M⁺+H].

The compound has a calculated Log POW of 1.68.

### Example 5

### Preparation of 2-({1-[2-(4-Phenyl-butyrylamino)-acetyl]-pyrrolidine-2-carbonyl}-amino)-propionic acid ethyl ester

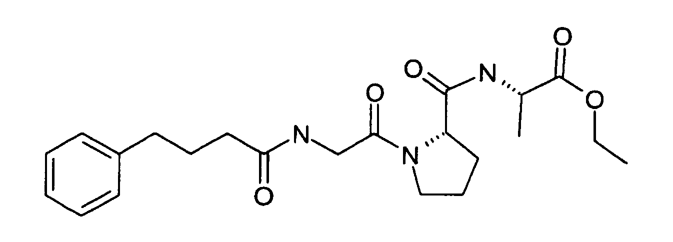

To EtOH (100 mL) was added acetyl chloride (484 mg, 6.2 mmol, 3.0 eq.). 2-{[1-(2-Amino-acetyl)-pyrrolidine-2-carbonyl]-amino}-propionic acid (500 mg, 2.1 mmol, 1.0 eq.) was added and the solution stirred at room temperature for 72 h. The solvent was evaporated under reduced pressure and the residue was twice coevaporated with pentane (50 mL) to yield 2-{[1-(2-Amino-acetyl)-pyrrolidine-2-carbonyl]-amino}-propionic acid ethyl ester hydrochloride (559 mg, quant.) as a white solid. - ¹H NMR (DMSO-d₆, 300 MHz) δ = 1.15-1.22 (m, 3H), 1.25-1.35 (m, 3H), 1.72-2.34 (m, 4H), 3.17-3.59 (m, 2H), 3.71-3.88 (m, 2H), 3.99-4.53 (m, 4H), 8.15-8.72 (m, 4H).

4-Phenyl-butyric acid (338 mg, 2.1 mmol, 1.0 eq.) was dissolved in DMF (20 mL) and CH₂Cl₂ (20 mL). HOBt (362 mg, 2.7 mmol, 1.3 eq.) and EDC (474 mg, 2.5 mmol, 1.2 eq.) were added and the solution stirred for 15 min at room temperature. 2-{[1-(2-Amino-acetyl)-pyrrolidine-2-carbonyl]-amino}-propionic acid ethyl ester hydrochloride (559 mg, 2.1 mmol, 1.0 eq.) and NEt₃ (313 mg, 3.1 mmol, 1.5 eq.) were added. The solution was stirred for 24 h at room temperature. The solvent was evaporated under educed pressure and the residue was taken up in ethyl acetate (75 mL) and water (30 mL). The aqueous layer was extracted with ethyl acetate (50 mL) and the combined organic layers were washed twice with an aqueous solution of 5 % HCl (50 mL), with a saturated aqueous solution of NaHCO₃ (50 mL), with a saturated aqueous solution of NaCl (50 mL) and then dried over Na₂SO₄. The solvent was evaporated under reduced pressure and the residue was purified by flash chromatography using CH₂Cl₂/MeOH (9:1) to yield 2-({1-[2-(4-Phenyl-butyrylamino)-acetyl]-pyrrolidine-2-carbonyl}-amino)-propionic acid ethyl ester (320 mg, 37 %) as a colorless solid. - R*_{f}* (ethyl acetate/hexane) = 0.50; ¹H NMR (CDCl₃, 300 MHz) δ = 1.12-1.42 (m, 6H), 1.80-2.42 (m, 8H), 2.52-2.61 (m, 2H), 3.30-4.56 (m, 7H), 6.41 (s, 1 H), 6.70-7.29 (m, 7H); ¹³C NMR (CDCl₃, 75.5 MHz) δ = 14.1, 18.2, 24.8, 27.1, 27.9, 35.2, 35.6, 42.1, 46.5, 48.4, 60.2, 61.4, 61.7, 77.2, 125.9, 128.4, 128.5, 141.5, 168.1, 170.4, 170.7, 172.8, 172.8; MS m/z = 418 (100) [M⁺+H].

The compound has a calculated Log POW of 1.59.

### Example 6

### Preparation of 1-{2-[2-(4-Phenyl-butyrylamino)-acetylamino]-propionyl}-pyrrolidine-2-carboxylic acid ethyl ester

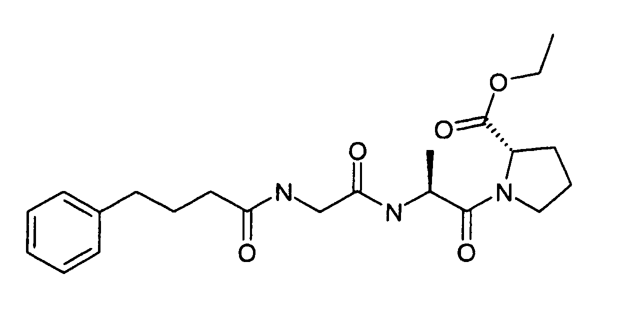

To EtOH (200 mL) was added acetyl chloride (1.3 g, 16.1 mmol, 3.0 eq.). 1-(2-Amino-propionyl)-pyrrolidine-2-carboxylic acid (1.0 g, 5.4 mmol, 1.0 eq.) was added and the solution stirred at room temperature for 48 h. The solvent was evaporated under reduced pressure to yield 1-(2-Amino-propionyl)-pyrrolidine-2-carboxylic acid ethyl ester hydrochloride (1.4 g, quant.) as a white solid. -¹H NMR (D₂O, 300 MHz) δ = 1.17 (t, *J* = 7.1 Hz, 3H), 1.45 (d, *J* = 7.0 Hz, 3H), 1.84-2.02 (m, 3H), 2.17-2.32 (m, 1H), 3.55-3.69 (m, 2H), 4.12 (q, *J* = 7.1 Hz, 2H), 4.28 (q, J = 7.1 Hz, 1 H), 4.37-4.44 (m, 1H).

(4-Phenyl-butyrylamino)-acetic acid (597 mg, 2.7 mmol, 1.0 eq.) was dissolved in DMF (10 mL) and CH₂Cl₂ (10 mL). HBTU (1.2 g, 3.0 mmol, 1.2 eq.), 1-(2-Amino-propionyl)-pyrrolidine-2-carboxylic acid ethyl ester hydrochloride (653 mg, 2.6 mmol, 1.0 eq.) and pyridine (0.2 mg, 3.0 mmol, 1.2 eq.) were added and the solution was stirred for 48 h at room temperature. The solvent was evaporated under educed pressure and the residue was taken up in ethyl acetate (50 mL) and water (50 mL). The aqueous layer was extracted twice with ethyl acetate (50 mL) and the combined organic layers were washed twice with an aqueous solution of 5 % HCl (75 mL), with a saturated aqueous solution of NaHCO₃ (75 mL), with a saturated aqueous solution of NaCl (75 mL) and then dried over Na₂SO₄. The solvent was evaporated under reduced pressure and the residue was purified by flash chromatography using ethyl acetate to yield 1-{2-[2-(4-Phenyl-butyrylamino)-acetylamino]-propionyl}-pyrrolidine-2-carboxylic acid ethyl ester (390 mg, 94 %) as a colorless oil. - R*_{f}* (ethyl acetate/hexane) = 0.56; ¹H NMR (CDCl₃, 300 MHz) δ = 1.19-1.31 (m, 4H), 1.97 (d, J = 7.7 Hz, 3H), 1.89-2.11 (m, 5H), 2.17-2.31 (m, 3H), 2.57-2.71 (m, 2H), 3.47-4.85 (m, 8H), 6.41-6.52 (m, 1H), 7.15-7.29 (m, 5H); ¹³C NMR (CDCl₃, 75.5 MHz) δ = 14.1, 17.7, 24.8, 26.9, 28.9, 35.1, 35.5, 42.7, 46.8, 46.9, 58.9. 61.1, 125.8, 128.3, 128.4, 141.5, 168.3, 171.0, 171.7, 173.0; MS m/z = 418 (100) [M⁺+H]. The compound has a calculated Log POW of 2.29.

### Example 7

### 1-{1-[2-(4-Phenyl-butyrylamino)-acetyl]-pyrrolidine-2-carbonyl}-pyrrolidine-2-carboxylic acid ethyl ester

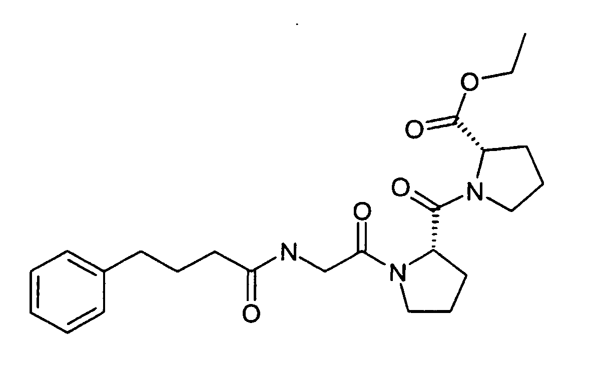

### Synthesis in analogy to example 6

The compound has a calculated Log POW of 2.18.
Molecular Weight =443.55
Exact Mass =443
Molecular Formula =C24H33N3O5
Molecular Composition =C 64.99% H 7.50% N 9.47% O 18.04%

### Example 8

### Anti-aging cream

| **O/W emulsion with trans-4-Hydroxy-L-Proline-4-phenylbutyric Acid Ethylester** | |
|---|---|
| **(Example 1)** | |
| | |
| **Ingredients** | **% (w/w)** |
| Glyceryl Myristate | 4.00 |
| Cetyl Alcohol | 2.00 |
| Steareth-2 | 2.00 |
| Steareth-21 | 2.00 |
| Isopropyl Myristate | 5.00 |
| Caprylic/Capric Triglyceride | 8.00 |
| BHT | 0.05 |
| Dimethicone | 2.00 |
| Phenoxyethanol & Methylparaben & Ethylparaben & Butylparaben & Propylparaben & Isobutylparaben | 0.80 |
| trans-4-Hydroxy-L-Proline-4-phenylbutyric Acid | 0.50 |
| Water | 69.05 |
| Xanthan Gum | 0.50 |
| Disodium EDETA | 0.10 |
| Propylene Glycol | 4.00 |

### Example 9

### Anti-aging cream

| **O/W emulsion with the compound of Example 2** | |
|---|---|
| | |

| **Ingredients** | **% (w/w)** |
|---|---|
| Glyceryl Myristate | 4.00 |
| Cetyl Alcohol | 2.00 |
| Steareth-2 | 2.00 |
| Steareth-21 | 2.00 |
| Isopropyl Myristate | 5.00 |
| Caprylic/Capric Triglyceride | 8.00 |
| BHT | 0.05 |
| Dimethicone | 2.00 |
| Phenoxyethanol & Methylparaben & Ethylparaben & Butylparaben & Propylparaben & Isobutylparaben | 0.80 |
| Compound of example 2 | 0.50 |
| Water | 69.05 |
| Xanthan Gum | 0.50 |
| Disodium EDETA | 0.10 |
| Propylene Glycol | 4.00 |

### Example 10

### Anti-aging cream

| **O/W emulsion with the compound of Example 4** | |
|---|---|
| | |

| **Ingredients** | **% (w/w)** |
|---|---|
| Glyceryl Myristate | 4.00 |
| Cetyl Alcohol | 2.00 |
| Steareth-2 | 2.00 |
| Steareth-21 | 2.00 |
| Isopropyl Myristate | 5.00 |
| Caprylic/Capric Triglyceride | 8.00 |
| BHT | 0.05 |
| Dimethicone | 2.00 |
| Phenoxyethanol & Methylparaben & Ethylparaben & Butylparaben & Propylparaben & Isobutylparaben | 0.80 |
| Compound of example 4 | 0.50 |
| Water | 69.05 |
| Xanthan Gum | 0.50 |
| Disodium EDETA | 0.10 |
| Propylene Glycol | 4.00 |

### Wrinkle reduction assay

The ability of the compounds and compositions of the present invention to reduce skin wrinkles can be assessed by profilometric methods described in "Skin topography measurement by interference fringe projection: a technical validation". (Lagarde J M; Rouvrais C; Black D; Diridollou S; Gall Y, Skin research and technology: official journal of International Society for Bioengineering and the Skin (ISBS) [and] International Society for Digital Imaging of Skin (ISDIS) [and] International Society for Skin Imaging (ISSI) (2001 May), 7(2), 112-21 or "Direct and non-direct measurement techniques for analysis of skin surface topography". Fischer T W; Wigger-Alberti W; Elsner P., Skin pharmacology and applied skin physiology (1999 Jan-Apr), 12(1-2), 1-11.

### Assessment of accelerated hair growth and protection hair growth

The ability of the compounds and compositions of the present invention to stimulate or protect hair growth can be investigated with a mouse model described for example in WO 9817273. Instead of using Cyclophosphamide (Neostar, Pharmacia) to damage hair follicle Mitomycin, or Methotrexate can be used. It is also possible to detect hair growth acceleration with newborn mice. They have a synchronized hair cycle and approximately after 3 weeks all hair follicles go into the telogen phase. Then the animals are treated and it is evaluated how fast and to what extend the hair is growing. Similar tests using *in vitro* or *in vivo* setups can also be found in J. Invest. Dermato. symposium proceedings 3rd Int. Meeting of Hair Research Societies, 8/1, p.39 - 45 (2003).

It also is possible to perform a clinical study including males suffering from alopecia using the TrichoScan analysis tool described in R. Hoffmann, J. Invest. Dermato. symposium proceedings 3rd Int. Meeting of Hair Research Societies, 8/1, p.109-115 (2003),

## Claims

1. Cosmetic composition comprising at least one compound represented by general formula (I) 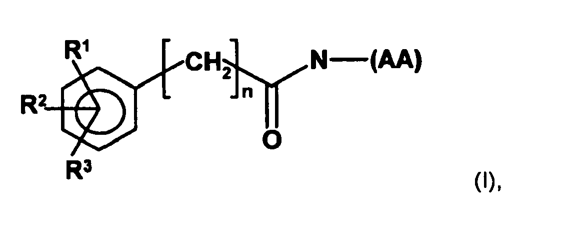 wherein R¹, R² and R³ are independently hydrogen, OR⁴, C₁-C₆ alkyl or C₂-C₆ alkenyl,
R⁴ is hydrogen, C₁-C₆ alkyl or C₂-C₆ alkenyl,
-N-(AA) represents the residue of an amino acid or of a peptide which is bonded over the N-terminus of the amino acid or the peptide and the peptide is composed of 2 to 6, that means 2, 3, 4, 5 or 6, amino acids, wherein the C-terminus of the amino acid or the peptide is optionally esterified with a C₁-C₁₆ hydrocarbon moiety and
n=3
and a cosmetically acceptable excipient or diluent, which composition is a topical composition.

2. Cosmetic composition according to claim 1, **characterized in that** R¹ and R² are both hydrogen and R³ is a residue -O-C₁-C₆ alkyl.

3. Cosmetic composition according to claim 1, **characterized in that** R¹, R² and R³ are hydrogen.

4. Cosmetic composition according to any of claims 1 to 3, wherein the C-terminus of the amino acid or the peptide is esterified with a C₁-C₁₆ alkyl residue.

5. Cosmetic composition according to any of claims 1 to 4, **characterized in that** -N-(AA) represents the residue of an amino acid selected from Glycine, α- or β-Alanine, Valine, Leucine, Isoleucine, Proline, Phenylalanine, Tryptophan, Methionine, Selenomethionine, Serine, Threonine, Cysteine, Hydroxyproline, Asparagin, Glutamine, Aspartic acid, Glutamic acid, Lysine, Hydroxylysine, Histidine, Arginine, Ornithine, Citrulline, Taurine, Sarcosine and Statine, Norleucine, Norvaline, or 2-N-Methylnorleucine

6. Cosmetic composition according to claim 5 where N-(AA) represents Hydroxyproline or an ester of Hydroxyproline.

7. Cosmetic composition of claim 6 where N-(AA) is Hydroxyproline-ethylester.

8. Cosmetic composition according to any of claims 1 to 4, **characterized in that** -N-(AA) represents the residue of a dipeptide.

9. Cosmetic composition according to any of claims 1 to 4, **characterized in that** -N-(AA) represents the residue of Carnosine, Balenine, Anserine, Glycinhydroxyproline or an ester of any of the above dipeptides.

10. Cosmetic composition according to any of claims 1 to 4, **characterized in that** -N-(AA) represents the residue of a tripeptide.

11. Cosmetic composition according to any of claims 1 to 10, wherein the compound of general formula (I) has a calculated log POW of 0 - 6.

12. Cosmetic composition according to any of claims 1 to 11, **characterized in that** the composition contains the compound of formula (I) in a concentration of 0.001 to 50 wt.-%, based on the weight of the composition.

13. Cosmetic composition according to claim 12, **characterized in that** the compound of formula (I) is present in a concentration of 0.01 to 1 wt.-%, based on the weight of the composition.

14. Use of a compound represented by general formula (I) as defined in any of claims 1 to 11 for the preparation of a composition for providing a cosmetic effect.

15. Use according to claim 14, wherein the cosmetic effect is treatment or prophylaxis of wrinkles or dry skin or sensitive skin or any symptoms caused by negative developments of the physiological homeostasis of healthy skin, promotion of hair growth, protection from hair loss, a thickening of the epidermis, anti-acne, the inhibition of senesence of skin cells, prevention or treatment of photodamage, prevention or treatment of oxidative stress phenomena, prevention or treatment of cellulite, prevention or treatment of pigmentation disorders and/or even the skin tone, prevention and treatment of disturbances in ceramide and lipid synthesis, prevention of excess sebum production, reduction of activities of matrix metallo proteases or other proteases in the skin, treatment and prevention of inflammatory skin conditions including atopic eczema, polymorphic light eruption, psoriasis, vertiligo, prevention and treatment of itchy or irritated skin.

## Patentansprüche

1. Kosmetische Zusammensetzung, umfassend mindestens eine Verbindung, dargestellt durch die allgemeine Formel (I) 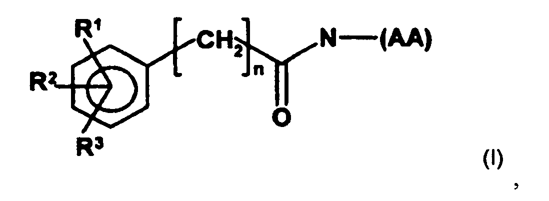 wobei R¹, R² und R³ unabhängig Wasserstoff, OR⁴, C₁-C₆-Alkyl oder C₂-C₆-Alkenyl sind,
R⁴ Wasserstoff, C₁-C₆-Alkyl oder C₂-C₆-Alkenyl ist,
-N-(AA) den Rest einer Aminosäure oder eines Peptids darstellt, der über den N-Terminus der Aminosäure oder des Peptids gebunden ist, und das Peptid aus 2 bis 6, das heißt 2, 3, 4, 5 oder 6, Aminosäuren besteht, wobei der C-Terminus der Aminosäure oder des Peptids gegebenenfalls verestert ist mit einer C₁-C₁₆-Kohlenwasserstoffkomponente und
n=3
und ein kosmetisch akzeptabler Hilfsstoff oder ein kosmetisch akzeptables Verdünnungsmittel, wobei die Zusammensetzung eine topische Zusammensetzung ist.

2. Kosmetische Zusammensetzung nach Anspruch 1, **dadurch gekennzeichnet, dass** R¹ und R² beide Wasserstoff sind und R³ ein Rest -O-C₁-C₆-Alkyl ist.

3. Kosmetische Zusammensetzung nach Anspruch 1, **dadurch gekennzeichnet, dass** R¹, R² und R³ Wasserstoff sind.

4. Kosmetische Zusammensetzung nach einem der Ansprüche 1 bis 3, wobei der C-Terminus der Aminosäure oder des Peptids mit einem C₁-C₁₆-Alkylrest verestert ist.

5. Kosmetische Zusammensetzung nach einem der Ansprüche 1 bis 4, **dadurch gekennzeichnet, dass** -N-(AA) den Rest einer Aminosäure, ausgewählt aus Glycin, α- oder β-Alanin, Valin, Leucin, Isoleucin, Prolin, Phenylalanin, Tryptophan, Methionin, Selenomethionin, Serin, Threonin, Cystein, Hydroxyprolin, Asparagin, Glutamin, Asparaginsäure, Glutaminsäure, Lysin, Hydroxylysin, Histidin, Arginin, Ornithin, Citrullin, Taurin, Sarcosin und Statin, Norleucin, Norvalin oder 2-N-Methylnorleucin, darstellt.

6. Kosmetische Zusammensetzung nach Anspruch 5, wobei -N-(AA) Hydroxyprolin oder einen Ester von Hydroxyprolin darstellt.

7. Kosmetische Zusammensetzung nach Anspruch 6, wobei -N-(AA) Hydroxyprolinethylester ist.

8. Kosmetische Zusammensetzung nach einem der Ansprüche 1 bis 4, **dadurch gekennzeichnet, dass** -N-(AA) den Rest eines Dipeptids darstellt.

9. Kosmetische Zusammensetzung nach einem der Ansprüche 1 bis 4, **dadurch gekennzeichnet, dass** -N-(AA) den Rest von Carnosin, Balenin, Anserin, Glycinhydroxyprolin oder einen Ester eines der obigen Dipeptide darstellt.

10. Kosmetische Zusammensetzung nach einem der Ansprüche 1 bis 4, **dadurch gekennzeichnet, dass** -N-(AA) den Rest eines Tripeptids darstellt.

11. Kosmetische Zusammensetzung nach einem der Ansprüche 1 bis 10, wobei die Verbindung der allgemeinen Formel (I) einen berechneten log-POW von 0 - 6 aufweist.

12. Kosmetische Zusammensetzung nach einem der Ansprüche 1 bis 11, **dadurch gekennzeichnet, dass** die Zusammensetzung die Verbindung der Formel (I) in einer Konzentration von 0,001 bis 50 Gew.-%, basierend auf dem Gewicht der Zusammensetzung, enthält.

13. Kosmetische Zusammensetzung nach Anspruch 12, **dadurch gekennzeichnet, dass** die Verbindung der Formel (I) in einer Konzentration von 0,01 bis 1 Gew.-%, basierend auf dem Gewicht der Zusammensetzung, vorliegt.

14. Verwendung einer Verbindung, dargestellt durch die allgemeine Formel (I), wie in einem der Ansprüche 1 bis 11 definiert, zur Herstellung einer Zusammensetzung für die Bereitstellung einer kosmetischen Wirkung.

15. Verwendung nach Anspruch 14, wobei die kosmetische Wirkung die Behandlung oder Prophylaxe von Falten oder trockener Haut oder empfindlicher Haut oder jeglicher Symptome, hervorgerufen durch negative Entwicklungen der physiologischen Stabilität gesunder Haut, die Förderung des Haarwachstums, der Schutz vor Haarausfall, eine Verdickung der Epidermis, Anti-Akne, die Verzögerung des Älterwerdens der Hautzellen, die Vorbeugung oder Behandlung von Lichtschädigung, die Vorbeugung oder Behandlung des oxidativen Stressphänomens, die Vorbeugung oder Behandlung von Cellulite, die Vorbeugung oder Behandlung von Pigmentstörungen und/oder sogar des Hauttons, die Vorbeugung und Behandlung von Störungen der Ceramid- und Lipidsynthese, die Vorbeugung von überschüssiger Talgproduktion, die Verringerung der Aktivitäten von Matrix-Metalloproteasen oder anderen Proteasen in der Haut, die Behandlung und Vorbeugung von entzündlichen Hautzuständen, einschließlich atopischer Dermatitis, polymorphes Lichterythem, Psoriasis, Vitiligo, die Vorbeugung und Behandlung von juckender oder gereizter Haut ist.

## Revendications

1. Composition cosmétique comprenant au moins un
composé représenté par la formule générale (I) 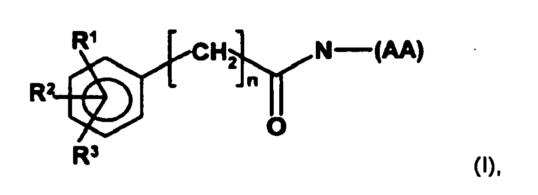 dans laquelle R¹, R² et R³ sont indépendamment un atome d'hydrogène, OR⁴, un groupe alkyle en C₁-C₆ ou alcényle en C₂-C₆,
R⁴ est un atome d'hydrogène, un groupe alkyle en C₁-C₆ ou alcényle en C₂-C₆,
-N-(AA) représente le résidu d'un acide aminé ou d'un peptide qui est lié par l'atome d'azote terminal de l'acide aminé ou du peptide et le peptide est constitué de 2 à 6, c'est-à-dire 2, 3, 4, 5 ou 6, acides aminés, où l'atome de carbone terminal de l'acide aminé ou du peptide est éventuellement estérifié par un groupement hydrocarboné en C₁-C₁₆ et n=3
et un excipient ou un diluant acceptable en cosmétique, composition qui est une composition locale.

2. Composition cosmétique selon la revendication 1, **caractérisée en ce que** R¹ et R² sont tous deux des atomes d'hydrogène et R³ est un résidu -O-alkyle en C₁-C₆.

3. Composition cosmétique selon la revendication 1, **caractérisée en ce que** R¹, R² et R³ sont des atomes d'hydrogène.

4. Composition cosmétique selon l'une quelconque des revendications 1 à 3, dans laquelle l'atome de carbone terminal de l'acide aminé ou du peptide est estérifié par un résidu alkyle en C₁-C₁₆.

5. Composition cosmétique selon l'une quelconque des revendications 1 à 4, **caractérisée en ce que** -N-(AA) représente le résidu d'un acide aminé choisi parmi la glycine, l'α- ou la β-alanine, la valine, la leucine, l'isoleucine, la proline, la phénylalanine, le tryptophane, la méthionine, la sélénométhionine, la sérine, la thréonine, la cystéine, l'hydroxyproline, l'asparagine, la glutamine, l'acide aspartique, l'acide glutamique, la lysine, l'hydroxylysine, l'histidine, l'arginine, l'ornithine, la citrulline, la taurine, la sarcosine et la statine, la norleucine, la norvaline ou la 2-N-méthylnorleucine.

6. Composition cosmétique selon la revendication 5, dans laquelle N-(AA) représente l'hydroxyproline ou un ester d'hydroxyproline.

7. Composition cosmétique selon la revendication 6, dans laquelle N-(AA) est l'hydroxyproline-éthylester.

8. Composition cosmétique selon l'une quelconque des revendications 1 à 4, **caractérisée en ce que** -N-(AA) représente le résidu d'un dipeptide.

9. Composition cosmétique selon l'une quelconque des revendications 1 à 4, **caractérisée en ce que** -N-(AA) représente le résidu de la carnosine, de la balénine, de l'ansérine, de la glycinhydroxyproline ou d'un ester de l'un quelconque des dipeptides ci-dessus.

10. Composition cosmétique selon l'une quelconque des revendications 1 à 4, **caractérisée en ce que** -N-(AA) représente le résidu d'un tripeptide.

11. Composition cosmétique selon l'une quelconque des revendications 1 à 10, dans laquelle le composé de formule générale (I) a un log POW calculé de 0 à 6.

12. Composition cosmétique selon l'une quelconque des revendications 1 à 11, **caractérisée en ce que** la composition contient le composé de formule (I) dans une concentration de 0,001 à 50 % en poids, par rapport au poids de la composition.

13. Composition cosmétique selon la revendication 12, **caractérisée en ce que** le composé de formule (I) est présent dans une concentration de 0,01 à 1 % en poids, par rapport au poids de la composition.

14. Utilisation d'un composé représenté par la formule générale (I) tel que défini dans l'une quelconque des revendications 1 à 11 pour la préparation d'une composition destinée à fournir un effet cosmétique.

15. Utilisation selon la revendication 14, dans laquelle l'effet cosmétique est le traitement ou la prophylaxie de rides ou d'une peau sèche ou d'une peau sensible ou d'un quelconque symptôme provoqué par des développements préjudiciables d'une homéostasie physiologique d'une peau saine, la promotion d'une croissance capillaire, la protection d'une perte de cheveux, un épaississement de l'épiderme, une action anti-acné, l'inhibition de la sénescence de cellules capillaires, la prévention ou le traitement de lésions dues à la lumière, la prévention ou le traitement d'un phénomène de stress oxydant, la prévention ou le traitement de la cellulite, la prévention ou le traitement de troubles de la pigmentation et/ou même du teint de la peau, la prévention et le traitement de troubles de la synthèse de céramides et de lipides, la prévention de la production de sébum en excès, la réduction des activités des métallo-protéases de matrice ou d'autres protéases dans la peau, le traitement et la prévention de maladies inflammatoires de la peau y compris l'eczéma local, la lucite, le psoriasis, le vertige, la prévention et le traitement d'une peau irritée ou qui démange.
